(19) Europäisches Patentamt
European Patent Office
Office européen des brevets

(11) Numéro de publication: **0 332 530**
**A1**

(12) # DEMANDE DE BREVET EUROPEEN

(21) Numéro de dépôt: **89400635.2**

(22) Date de dépôt: **07.03.89**

(51) Int. Cl.4: **C 08 G 69/26**
A 61 K 9/22, A 61 L 17/00,
A 61 L 25/00

(30) Priorité: **08.03.88 FR 8802956**

(43) Date de publication de la demande:
**13.09.89 Bulletin 89/37**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Boustta, Mamfoud**
**90, Avenue Porde des Champs**
**F-76000 Rouen (FR)**

**Huguet, Jovanka**
**8, Allée Primevères Hénouville**
**F-76840 St Martin Boscherville (FR)**

**Vert, Michel**
**3, rue de la Vatine**
**F-76130 Mont Saint Aignan (FR)**

(74) Mandataire: **Le Guen, Gérard et al**
**CABINET LAVOIX 2, place d'Estienne d'Orves**
**F-75441 Paris Cédex 09 (FR)**

Revendications pour l'Etat contractant suivant: ES.

(54) **Polymères d'acide citrique et de diamines, leur procédé de préparation et leurs applications notamment comme vecteurs de médicaments.**

(57) La présente invention a pour objet des polyamides résultant de la condensation de l'acide citrique, par l'intermédiaire des groupes carboxyliques portés par les carbones en position 1 et 3, avec des diamines.

Ces polyamides sont utilisables pour la préparation de formes à libération contrôlée de médicaments.

EP 0 332 530 A1

## Description

### Polymères d'acide citrique et de diamines, leur procédé de préparation et leurs applications notamment comme vecteurs de médicaments

La présente invention concerne des polymères hydrophiles constitués par des polyamides résultant de la condensation de l'acide citrique, par l'intermédiaire des groupes carboxyliques portés par les carbones en position 1 et 3, c'est-à-dire en position béta par rapport au groupe hydroxy, avec des diamines, ainsi que leur procédé de préparation et l'application de ces polymères biodégradables comme vecteurs ou comme réservoirs de médicaments permettant la libération progressive contrôlée de ceux-ci dans l'organisme, comme sutures ou ligatures ou comme prothèses chirurgicales pour la reconstitution et le soutien des tissus vasculaires, des ligaments ou des os lésés et comme adhésifs chirurgicaux.

Des polyamides porteurs de fonctions hydrophiles, ont déjà été décrits. On peut citer ceux préparés par condensation de l'acide L-tartrique avec l'hexaméthylène diamine décrits par Minoura et coll. dans J. Polym. Sc. Part-A-1 5 p. 2441 (1967) ou par N. Ogata et coll. dans J. Polym. Sc. 13 p. 1793 (1975); les premiers sont solubles dans l'eau mais de faible masse moléculaire tandis que les seconds sont insolubles dans l'eau. Des polyamides hydrosolubles dérivés de L-lysine et de diacides aliphatiques et aromatiques ont aussi été préparés par polycondensation interfaciale, comme mentionné dans Makromol. Chem. 109 p. 239 (1967) et J. Polym. Sc. Part A-1 9 p. 2413 (1971) ou par polycondensation en solution comme mentionné dans Makromol. Chem. 186 p. 939 (1985).

Il n'a cependant jamais été décrit de polyamide dérivé de l'acide citrique; pourtant, la présence dans ce monomère de trois groupes carboxyliques et d'un groupe hydroxyle permet d'obtenir un polymère porteur d'au moins deux fonctions hydrophiles, ce qui lui confère une certaine solubilité dans l'eau, quel que soit le pH du milieu, et dans les solvants polaires, tandis que la présence d'un groupe carboxylique libre ou engagé dans une liaison intramoléculaire labile confère au polymère une bonne réactivité, qui peut être mise à profit pour fixer sur le squelette du polymère linéaire, par liaison covalente ou ionique, diverses molécules, et par exemple des médicaments, ou pour le réticuler si l'on veut diminuer sa solubilité intrinséque et augmenter sa résistance mécanique.

Les polymères de l'invention répondent en particulier à la formule

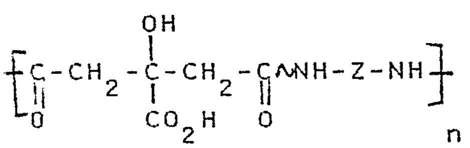

I

dans laquelle n représente un nombre entier inférieur à 1000, et de préférence compris entre 20 et 300, et Z représente une chaîne aliphatique linéaire ou ramifiée, ayant de 2 à 10 atomes de carbone, et de préféence de 2 à 8 atomes de carbone, pouvant comporter des substituants hydrophiles, tels que OH et COOH, ou dans laquelle peuvent être intercalés des atomes d'oxygène ou de soufre, ou Z représente une chaîne aryle, telle que $-C_6H_4-$, ou alkylaryle telle que $-CH_2-C_6H_4-CH_2-$ ou $-CH_2-C_6-H_4-$.
et   représente les deux types d'enchaînement lorsque Z est dissymétrique.

En effet, dans ce cas, le polymère est constituée de 2 types d'unités résultant de la condensation de l'une ou l'autre fonction amine de la diamine avec la fonction carboxylique libre du polymère en croissance.

Pour être utilisés en thérapeutique, notam ment dans des formes à libération contrôlée de médicaments, les polymères doivent de préférence être bio-dégradables et se dégrader en donnant des molécules non toxiques pour l'organisme et facilement éliminées; on sait que les polyamides linéaires et aliphatiques, comme le nylon®, sont peu sensibles à l'hydrolyse in vivo; par contre, les polycitramides de l'invention sont, du fait de leur hydrophilie et de leur hydrosolubilité, hydrolysés sans difficulté in vivo, même en l'absence de protéases, en libérant de l'acide citrique, métabolite du cycle de Krebs, qui sera dégradé et éliminé par les voies métaboliques naturelles, sans risque d'accumulation ou de formation de produits toxiques. Dans le cas où la diamine monomère est une molécule présente naturellement dans l'organisme, la totalité des produits de biodégradation est éliminée de l'organisme par les voies métaboliques naturelles et l'on dit que le polymère est biorésorbable.

Les diamines utilisées dans la préparation des polymères de l'invention, peuvent être des diamines simples aliphatiques ou aromatiques de préférence symétriques, telles que l'éthylènediamine, l'hexaméthylène-diamine, la dioxa-3,6 octyl-diamine-1,8, la cystamine ou les phénylénediamines.

Les diamines peuvent aussi comporter d'autres groupes hydrophiles libres, par exemple des groupes hydroxyle ou carboxyle, comme dans l'acide diaminopimélique; dans ce cas, les groupements susceptibles de réagir au cours de la polycondensation doivent être protégés, préalablement à la polymérisation.

Surtout lorsque les polymères de l'invention sont destinés à des applications pharmaceutique, on préfère les diamines naturellement présentes dans l'organisme telles que la cystamine, la L-ornithine, la L-cystine, et tout particulièrement la L-lysine; on peut aussi utiliser les stéréoisomères dextrogyre de ces amines, ou leur

mélange racémique.

Les copolymères résultant de la polycondensation de l'acide citrique avec au moins deux diamines différentes sont aussi compris dans l'invention.

Un objet préféré de l'invention est le polymère résultant de la condensation de l'acide citrique, dont les fonctions hydroxyle et acide carboxylique portées par le carbone central ont été correctement protégées pour la polymérisation par condensation, avec la L-lysine dont la fonction acide carboxylique a été correctement protégée pour la polymérisation.

Les polymères ainsi obtenus, lorsque les groupes protecteurs ont été éliminés, sont hydrophiles, et plus ou moins solubles selon leur masse moléculaire, dans l'eau et les solvants miscibles à l'eau comme les solvants organiques aprotiques polaires et les solvant hydroxylés; la solubilité dans les alcools est particulèrement utile lors de la préparation des formes pharmaceutiques et il faut noter que la plupart des polymères résultant de la condensation des alphaaminoacides, dont l'utilisation comme polymères biodégradables est actuellement très étudiée, ne sont eux, en général solubles que dans des solvants peu employés en pharmacie industrielle.

Les polymères préférés de l'invention ne sont pas toxiques et sont dégradés in vivo par hydrolyse simple ou éventuellement enzymatique, en libérant des composés atoxiques, naturellement présents dans l'organisme, l'acide citrique et la L-lysine.

Un autre objet de l'invention est le procédé de préparation de ces polycitramides. Il consiste essentiellement à effectuer la polycondensation entre une diamine et l'acide citrique, dont les groupes OH et $CO_2H$ portés par le carbone en position 2 sont protégés et à libérer les groupes protégés.

La protection des groupes OH et $CO_2H$ portés par le carbone en position 2 doit être sélective, c'est-à-dire que les autres groupes $CO_2H$ de l'acide citrique doivent rester libres; en outre les groupes protégés doivent être stables, ou sensiblement stables, dans les conditions de la réaction de polycondensation, mais doivent pouvoir être libérés après la polymérisation sans décomposition du squelette du polyamide.

On préfère protéger simultanément les fonctions OH et $CO_2H$ en préparant un dérivé de type dioxolanne-1,3 one-4 de formule d par action d'un aldéhyde sur l'acide citrique selon le schéma réactionnel :

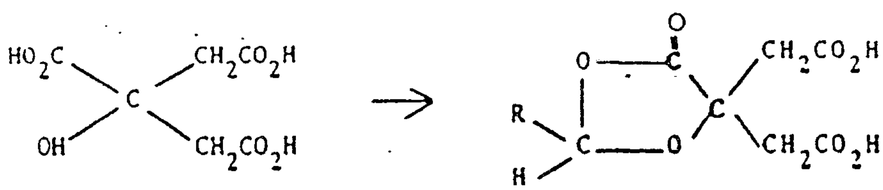

d

Les produits de condensation de l'acide citrique avec le formaldéhyde (R = H), avec un aldéhyde aliphatique (R = alkyle en $C_1$ à $C_4$), avec l'aldéhyde trichloracétique (R = $CCl_3$) et avec le benzaldéhyde (R = $C_6H_5$) sont des composés connus, et l'on sait qu'ils sont hydrolysables soit en milieu acide soit en milieu basique, en régénérant l'acide citrique de départ.

On a maintenant trouvé que l'acide citrique pouvait aussi être régénéré par hydrogénation catalytique des dioxolannes de formule d, ce qui permet la déprotection simultanée des unités citriques et diaminées lorsque celles-ci portent certaines fonctions réactives qui ont dû être protégées.

Il est nécessaire pour effectuer la condensation polymérisante, d'activer les fonctions acides et/ou les fonctions amines. On peut introduire dans une solution organique du diacide et de la diamine, un réactif de couplage, tel que le dicyclohexylcarbodiimide, ou le N,N'-carbonyl-diimidazole, mais on préfère activer préalablement, de façon classique, les acides sous forme de chlorures d'acides; pour cela, on peut soumettre l'acide citrique protégé, à l'action du chlorure de thionyle, en excès, en présence ou non d'un tiers solvant. On peut aussi activer préalablement les fonctions amines par substitution de celles-ci par le groupe triméthylsilyle ou par tout autre agent classique. D'autres réactifs de couplage, tels que ceux mis en oeuvre en synthèse peptidique, peuvent aussi être avantageusement utilisés pour la réaction de polycondensation.

La polymérisation peut être effectuée de façon classique en solution ou par polycondensation interfaciale, cette dernière méthode étant applicable avec des composés très réactifs, comme les chlorures d'acide, de préférence à une température voisine de la température ambiante ou même inférieure.

Le spécialiste sera à même de choisir les conditions de polymérisation les mieux adaptées pour obtenir des polymères ayant des caractéristiques spécifiques de masse moléculaire, en se référant notamment aux procédés préconisés pour la préparation des polyamides, et en tenant compte de l'instabilité du groupe dioxolanne protecteur de l'acide citrique en milieu basique aqueux. La polycondensation du dichlorure d'acide avec une diamine sera effectuée de façon connue en présence d'une base soluble qui captera l'acide chlorhydrique libéré; dans les polymérisations en solution, on introduira en général une amine tertiaire, comme la triéthylamine ou la pyridine, tandis qu'en polymérisation interfaciale on pourra introduire un carbonate alcalin, hydrosoluble.

Le procédé de libération des groupes ayant été protégés avant la polycondensation sera fonction de la méthode de protection choisie. Si l'acide citrique a été protégé sous forme de dioxolanne de formule d, les groupes OH et $CO_2H$ peuvent être régénérés par action en milieu aqueux ou hydroalcoolique d'un acide ou

d'une base, à température ambiante, sans dégradation du squelette polyamide.

Dans le cas où la diamine porte des fonctions réactives, autres que les deux groupes $NH_2$ condensables, le procédé de l'invention comportera en outre une étape de protection de ces fonctions avant la polycondensation, ainsi qu'une étape de libération de ces groupes protégés présents dans le polymère; cette dernière pourra être simultanée à celle de libération des OH et $CO_2H$ de l'acide citrique condensé. Il est évidemment nécessaire que ces groupes protecteurs puissant être éliminés sans dégradation du squelette du polymère et on pourra par exemple bloquer les fonctions hydroxyle de la diamine sous forme d'esters ou de carbonates et les fonctions carboxylique sous forme d'esters benzyliques; ce dernier groupe protecteur est préféré car il permet de régénérer l'acide par hydrogénation catalytique ou par hydrolyse, dans des conditions douces, alors que les esters d'alcools alphatiques linéaires ne sont hydrolysés qu'en milieu acide ou basique forts, dans des conditions où la chaîne polyamide serait aussi dégradée, au moins partiellement.

On a constaté que le produit obtenu par polycondensation interfaciale du dichlorure de l'acide citrique, protégé sous forme d'un dioxolanne de formule d, avec une diamine n'était pas constitué des seules séquences attendues, c'est-à-dire ne répondant pas uniquement aux formules $II_a$ et$II_b$, si l'on prend comme exemple de diamine la lysine, dont la fonction acide est protégée sous forme d'ester benzylique

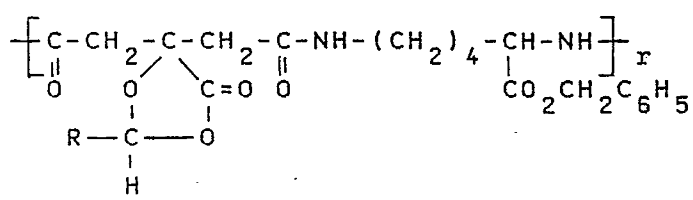

$II_a$

et

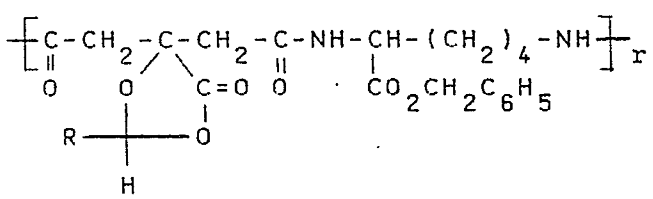

$II_b$

correspondant aux deux possibilités d'enchaînement de cette diamine dissymétrique, formules que l'on peut aussi noter sous forme condensée :

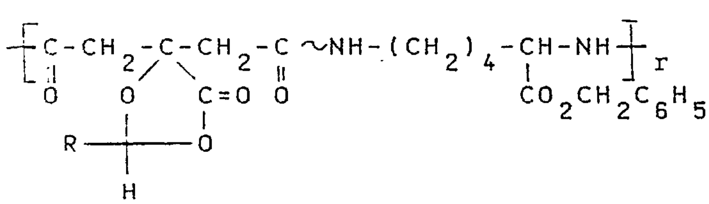

mais comportait aussi des séquences ayant des groupes imides cycliques résultant d'une réaction secondaire intramoléculaire du groupe dioxolanne, de telle sorte que le polycondensat brut répond à la formule III condensée :

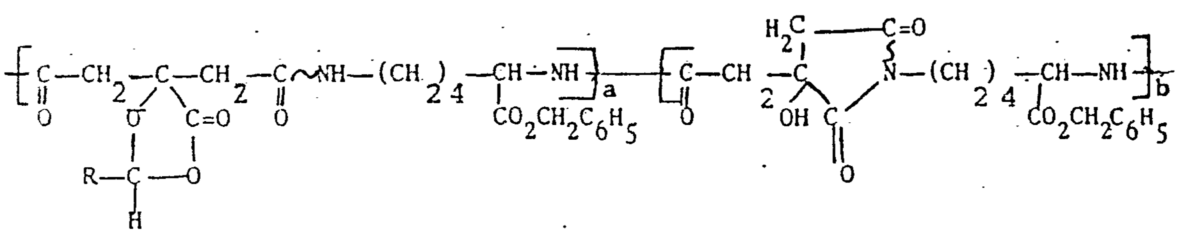

dans laquelle a et b sont des nombres entiers, et le signe ∿ traduit les diverses possibilités d'enchaînement dues à la dissymétrie de l'amine de départ.

La proportion de séquences à groupe imide est fonction de la nature du substituant R du dioxolanne et des conditions de la polycondensation, notamment du solvant.

Les copolymères de formule III sont un objet de l'invention; ils peuvent être utilisés comme polymères biodégradables, ou comme intermédiaire de synthèse des polymères de formule IV et V.

L'hydrogénation catalytique du polymère de formule III libère les fonctions acides carboxyliques des esters benzyliques et les fonctions hydroxyle et carboxyle engagées dans le cycle dioxolanne, et l'on obtient alors un polymère de formule IV, condensée :

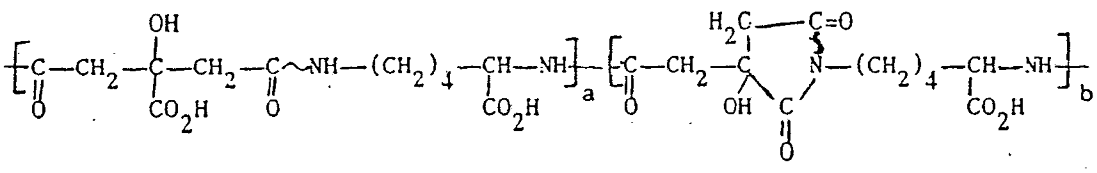

dans laquelle a et b sont des nombres entiers.

L'hydrogénolyse des fonctions esters pour obtenir le polymère est parfois difficile à rendre complète et on peut si nécessaire hydrolyser les fonctions esters restant en fin d'hydrogénation par action d'une solution acide, comme une solution d'acide chlorhydrique dans un mélange de diméthylformamide et d'eau.

Un autre objet de l'invention est constitué par le copolymère de formule IV, ainsi que par les polymères analogues préparés à partir d'autres diamines que la lysine de formule IV bis

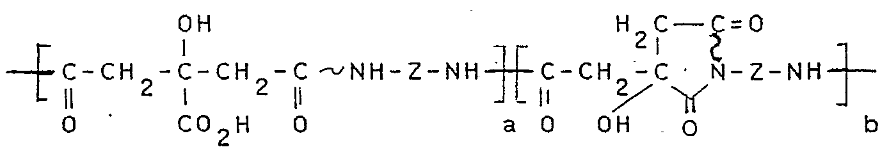

Par action d'une base en milieu aqueux, le cycle imide des polymères s'ouvre par coupure de l'une ou l'autre des liaisons carbonyle-azote pour donner après acidification, des copolymères linéaires, comportant deux types de séquences distinctes non cycliques à répartition statistique, répondant à la formule V :

$A_p - B_q$

dans laquelle A représente :

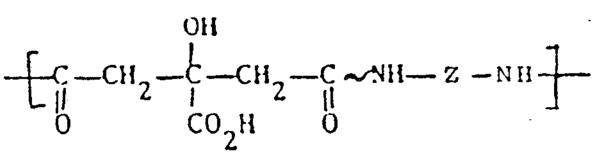

B représente :

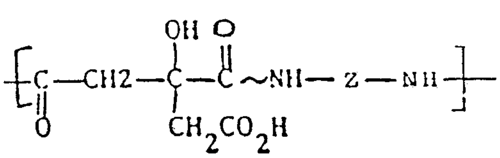

Z ayant la même signification que précédemment, et p et q représentent des nombres entiers, et sont tels que $p+q = a+b$ et $p > a$ et ∿ à la même signification que précédemment.

Les composés de formule V sont un autre objet de l'invention.

Dans le cas où les polymères de l'invention sont préparés à partir d'une diamine symétrique, on obtient des produits de structure plus régulière, répondant à la formule I dans laquelle ∿ est -, puisqu'il n'y a plus d'isomères dus aux enchaînements tête à tête et tête à queue.

Enfin, un autre objet de l'invention est l'application des polycitramides biodégradables et dans certains cas biorésorbables de formule I, III, IV et V, ainsi que de leurs sels avec des bases pharmaceutiquement acceptables, comme réservoirs ou vecteurs de médicaments dans des formes pharmaceutiques à libération contrôlée, comme sutures ou ligatures chirurgicales, comme prothèses chirurgicales, ou comme adhésifs.

Les principes actifs pourront être fixés par liaison covalente ou ionique aux polymères de l'invention pour donner des prodrogues macromoléculaires ou être intimement mélangés au polymère par des procédés classiques ou absorbés sur le polymère se présentant sous forme de billes, de feuilles, de batonnets ou d'éléments microporeux, ou encore incorporés dans des systèmes agrégés du polymère, tels que des microémulsions macromoléculaires; ils pourront aussi être enveloppés par le polymère notamment pour former des capsules ou des microcapsules, préparées selon des techniques classiques.

Des médicaments à effet curatif ou préventif des maladies, ou modifiant certaines fonctions biologiques pourront être formulés de façon classique avec les polymères de l'invention.

Les formes pharmaceutiques seront adaptées au mode d'administration du médicament, par voie orale ou nasale, par injection intraveineuse ou intramusculaire ou par implantation sous-cutanée.

Les polymères selon l'invention ne sont pas toxiques puisqu'injectés chez la souris en intrapéritonéal ou en intramusculaire à 700 mg/kg, il n'y a ni mort ni manifestation apparente de toxicité, tout comme chez le rat en intraveineux à 250 mg/kg.

Par ailleurs, implantés par voie sous-cutanée au niveau du flanc de rats, la tolérance locale des produits est excellente, malgré, parfois, une très légère réaction fibreuse.

Dans ce qui suit, on décrit des exemples de mise en oeuvre de l'invention.

Exemple 1 : Polycondensation interfaciale du dichlorure de citrobenzal et de l'ester benzylique de la L-lysine.

a) disel d'acide p-toluène sulfonique du L-lysinate de benzyle

30 g de L-lysine ($20,5.10^{-2}$ mole) et 70 ml d'alcool benzylique distillé ($67,6.10^{-2}$ mole) sont introduits dans un extracteur type Dean-Stark; on y ajoute 88,5 g d'acide paratoluène sulfonique monohydraté ($46,5.10^{-2}$ mole) et 300 ml de benzène. Le mélange est amené à ébullition puis à reflux jusqu'à élimination totale de l'eau formée par la réaction. La solution est transvasée dans un bécher dans lequel on ajoute 100 ml de méthanol. La précipitation du sel se fait par addition de 500 ml d'éther éthylique à température ambiante. Le précipité est repris par dissolution à chaud dans 300 ml de méthanol et reprécipité par l'éther éthylique. On récupère 100 g du N,N' ditosylate du L-lysinate de benzyle; Rdt = 84% F = 163°C.

b) Citrobenzal (composé de formule d,R = $C_6H_5$)

78 g d'acide citrique (0,41 mole) et 90 g de benzaldéhyde sont introduits dans un ballon tricol muni d'une agitation mécanique. Le milieu est porté à 50°C environ et on ajoute 42,5 g de$P_2O_5$ en petites quantités de façon à maintenir la température entre 75 et 80°C. L'anhydride phosphorique rougit et noircit au contact du mélange. En fin d'addition, le mélange prend une coloration noirâtre. L'agitation est prolongée pendant 90 minutes à une température voisine de 80°C. En fin de réaction, on verse l'huile noirâtre encore chaude dans un mortier contenant de la glace pilée; elle se solidifie au contact de la glace. Le mélange est neutralisé par addition KOH 4N jusqu'à pH 8, tout en le pilant. Le benzaldéhyde en excès est extrait par l'acétate d'éthyle. La solution aqueuse noirâtre est refroidie à 0°C et acidifiée par HCl concentré. Le citrobenzal se sépare au fond sous forme d'huile. Il est extrait à l'éther éthylique. La solution éthérée est lavée à l'eau jusqu'à pH neutre, séchée sur Mg $SO_4$ anhydre et évaporée. On obtient environ 70 g de solide blanc, lequel est dissous à chaud dans 300 ml d'acétate d'éthyle. Après refroidissement de la solution jaune, on ajoute 150 ml d'éther de pétrole, qui provoque la précipitation du citrobenzal en cristaux blancs. On récupère 51,5 g de cristaux; Rdt = 45%. F = 185°C ($CH_3COOC_2H_5$/éther pétrole). Spectres IR et RMN conformes.

c) Dichlorure de citrobenzal

14 g du diacide obtenu selon b) et 40 ml de $SOCl_2$ (préalablement distillé en présence de triphénylphosphite) sont chauffés dans un ballon muni d'un réfrigérant et d'une garde au chlorure de calcium. Sous agitation, le milieu réactionnel est chauffé à reflux jusqu'à dissolution complète du diacide (2 heures environ). Le reflux est ensuite maintenu pendant 15 minutes. En fin de réaction, le chlorure de thionyle en excès est éliminé sous pression réduite. On ajoute 30 ml de benzène ou de toluène au résidu d'évaporation; le mélange est filtré. La solution est chauffée en présence de charbon végétal, puis filtrée et évaporée à sec.

Le résidu est dissous dans 20 ml de benzène et reprécipité par l'éther de pétrole. On obtient 9,4 g de dichlorure de citrobenzal sous forme d'aiguilles blanches, Rdt de l'ordre de 60%.

F = 82°C (toluène/n-hexane).

Ce produit est facilement hydrolysé par l'eau et se transforme spontanément en anhydride cyclique; il doit donc être utilisé extemporanément.

d) Polycondensation

7,6 g de N,N ditosylate de l'ester benzylique de la L-lysine ($13,02.10^{-3}$ mole), 7,45 g de carbonate de sodium décahydraté ($26,04.10^{-3}$ mole) nécessaires pour neutraliser les fonctions acides libérées et 0,5 g de dodécylsulfate de sodium (MERCK) (10% en poids par rapport au dichlorure d'acide) sont dissous dans un mélange eau-benzène (60 $cm^3$ , V/V, 66/33) sous forte agitation dans un mixeur de laboratoire.

Sous agitation vigoureuse (12 à 15000 tours par minute), on introduit simultanément 4,54 g de dichlorure de citrobenzal (14,32.$10^{-3}$ mole) dissous dans 40 $cm^3$ de benzène et 8,2 g de carbonate de sodium (28,64.$10^{-3}$ mole) dissous dans 20 $cm^3$ d'eau, de telle façon que 90% en volume de chaque solution soient ajoutés en 6 minutes. On laisse sous agitation pendant 1 à 2 minutes, puis on additionne les 10% des solutions restantes en 1 minute et on maintient l'agitation pendant 2 minutes. La réaction démarre à température ambiante pour se terminer vers 45-50°C. Le polymère précipite dans le milieu après une minute d'addition. Le mélange réactionnel vire au jaune à mi-temps de réaction. A la fin de la réaction, le milieu est acidifié par de l'acide chlorhydrique dilué jusqu'à pH voisin de 2. Le polycondensat est lavé à l'eau jusqu'à pH neutre, séché sous vide à une température voisine de 50°C et pesé. On récupère 5,1 g de polymère de formule III (Rdt = 83%). (R = $C_6H_5$).

Ce polymère est un solide jaune, soluble dans l'acétone, les solvants chlorés tels que $CHCl_3$,-$CH_2Cl_2$, le dioxanne et le tétrahydrofuranne. Il est par contre insoluble dans le benzène, le toluène, le méthanol, l'éthanol et l'eau.

La masse moléculaire moyenne est 24000 (mesure effectuée par chromatographie de perméation de gel, en solution dans le dioxanne, avec des étalons de polystyrène).

Exemple 2 : polycondensation interfaciale du dichlorure de citrochloral et de l'ester benzylique de la L-lysine.

a) citrochloral (composé de formule d où R = $CCl_3$)

77 g d'acide citrique (0,4 mole) et 99,3 g de chloral anhydre (0,67 mole) sont introduits sous azote dans un ballon tricol muni d'une agitation mécanique, d'un réfrigérant, et d'une ampoule à brome. L'ensemble est refroidi à 0°C par un bain de glace. Sous agitation, on ajoute en 90 minutes environ, 120 ml d'acide sulfurique concentré en maintenant la température entre 0 et 5°C par ajout de glace. L'agitation est poursuivie pendant 2 heures à 0°C puis le mélange réactionnel est ramené à température ambiante et laissé sous agitation pendant 10 heures environ. En fin de réaction, le mélange prend l'aspect d'une pâte blanche que l'on verse dans un bécher contenant de la glace pilée. Le solide blanc est extrait par l'acétate d'éthyle et l'éther éthylique. La phase organique est lavée à l'eau jusqu'à pH neutre, séchée sur sulfate de sodium anhydre et évaporée à sec jusqu'à obtention d'une huile visqueuse rosâtre. Cette huile est dissoute à chaud dans 300 ml de chloroforme. Le citrochloral précipite à froid sous la forme de poudre blanche. Le précipité est repris par 350 ml d'acétate d'éthyle bouillant. L'addition progressive d'éther de pétrole à la solution jaune refroidie à température ambiante, provoque la précipitation de cristaux blancs. On récupère 96,5 g de citrochloral; Rdt = 75%. F = 163°C ($CH_3COOC_2H_5$/éther de pétrole).

b) Dichlorure de citrochloral

Il est préparé en appliquant le mode opératoire décrit à l'exemple 1-c. Toutefois, le résidu d'évaporation est dissous dans 20 ml de dichloroéthane-1,2, au lieu de benzène.

On obtient 9,5 g de dichlorure, à partir de 14 g de diacide, sous forme d'aiguilles blanches. F = 79-80°C ($CCl_4$/éther de pétrole).

c) Polycondensation interfaciale avec l'ester benzylique de la L-lysine

8,9 g de N,N-ditosylate de l'ester benzylique de la L-lysine (15,2.$10^{-3}$ mole), 8,71 g de car-bonate de sodium décahydraté (30,4.$10^{-3}$ mole) et 0,7 g de dodécylsulfate de sodium sont dissous sous forte agitation dans un mélange contenant 20 $cm^3$ d'eau, 10 $cm^3$ de dichlorométhane, 10 $cm^3$ de dichloro-1,2 éthane et 30 g de glace.

On introduit simultanément 6 g de dichlorure de citrochloral (16,7.$10^{-3}$ mole) dissous dans 500 $cm^3$ du mélange de solvants chlorés choisis (V/V-50/50) et 9,58 g de carbonate de sodium (33,4.$10^{-3}$ mole) dissous dans 20 $cm^3$ d'eau. Le mode opératoire est semblable au précédent.

En fin de réaction, on recueille la phase organique contenant le polycondensat brut. La phase aqueuse est acidifiée et extraite par 50 ml de dichlorométhane (2 fois). Les solutions organiques sont rassemblées, lavées à l'eau et séchées sur sulfate de magnésium. Le polycondensat, récupéré après évaporation des solvants organiques, est séché sous vide à 50°C. On récupère 7 g de polymère brut de formule III (R = $CCl_3$) (Rdt = 87%).

Le polymère brut de formule III (R = $CCl_3$) a des solubilités comparables à celles du polymère obtenu à l'exemple 1; sa masse moléculaire moyenne est 20000.

Les masses moléculaires des polymères sont fonction des conditions de polycondensation, de la température finale et des solvants utilisés : dans un mélange benzène/eau (50/50 - v/v), la polycondensation donne un polymère de masse moléculaire 20000, tandis qu'elle n'est que 10000 dans un mélange benzène/glace/eau (50/20/30) et 14000 dans un mélange chlorure de méthylène/dichloréthane/glace/eau (25/25/20/30).

Exemple 3 : Préparation du polymère de formule IV

A 5 g de copolycondensat brut obtenus aux exemples 1 ou 2 dissous dans 100 $cm^3$ de diméthylformamide (DMF), on ajoute 1 g de charbon palladié (10% de palladium activé sur charbon). Le milieu est maintenu à 60°C pendant 24 heures, c'est-à-dire jusqu'à ce qu'il n'y ait plus de consommation d'hydrogène.

Après filtration de la solution, le DMF est évaporé sous pression réduite à 50°C. Le copolymère est solubilisé dans 10 $cm^3$ de méthanol puis précipité par environ 100 $cm^3$ d'éther diéthylique. Le polymère est

récupéré sous la forme d'une pâte qui est séchée à l'étuve sous vide à 50°C pendant une nuit. On obtient 4,12 g de copolymère brunâtre de formule IV sous forme acide (Rdt = 84%).

Dans le cas du polycondensat de l'exemple 2, l'hydrogénolyse n'est pas totale après 24 heures de réaction. Il reste environ 6 à 10% de groupes ester benzylique fixés sur la lysine (déterminé par RMN du proton); ils peuvent être éliminés en reprenant l'hydrogénation après avoir introduit dans le milieu de nouveau 0,5 g charbon palladié ou par hydrolyse, 2 heures à température ambiante dans un mélange DMF/HCl 4N (60/40 - v/v). A la fin de l'opération, la solution est neutralisé par addition d'une solution aqueuse de NaOH 4N et dialysée contre de l'eau à travers un boyau de seuil de coupure 6000 à 8000, tel que celui commercialisé par Visking USA sous la référence 24012. Le rétentat débarrassé des solvants organiques, des sels et des oligomères est lyophilisé pour donner le sel de sodium du composé de formule IV sous forme d'une poudre brune, soluble dans l'eau. Le spectre IR (pastilles de KBr) de ce sel présente entre 1800 et 1500 $cm^{-1}$ cinq bandes, correspondant aux vibrations caractéristiques des carbonyles d'un imide (1780 $cm^{-1}$ et 1710 $cm^{-1}$) d'amide (1650 $cm^{-1}$ et 1550 $cm^{-1}$) et d'un groupe carboxylique salifié (1600 $cm^{-1}$). Le spectre RMN$^{13}$C dans $D_2O$ (référence externe pyridine) présente un pic dédoublé, pour le carbone quaternaire de l'unité citrique, dont l'un à 73,1 ppm, correspond à un carbone inclu dans un cycle imide et l'autre à 75,2 ppm, portant à un carbone les groupes OH et $CO_2H$ libérés. La proportion des groupes imides, estimée d'après les surfaces relatives des deux pics, est de 90% environ.

Le copolymère acide est libéré de son sel par percolation d'une solution aqueuse du sel sur une résine échangeuse de cations sous forme $H^+$, telle qu'une Duolite® C20MB, et isolé par lyophilisation. C'est un solide blanc, insoluble dans l'eau, les solvants chlorés, aromatiques, le tétrahydrofuranne et le dioxanne mais soluble dans le méthanol, le diméthylformamide et le diméthylsulfoxyde.

Lorsque l'hydrogénation catalytique du polycondensat brut est effectuée dans le mélange dioxanne/éthanol (50/50-V/V) au lieu du DMF, l'hydrolyse des groupes esters benzyliques est totale mais une partie des fonctions acide ainsi libérées est transformée en ester éthylique.

Exemple 4 : Prépration du copolymère de formule V par hydrolyse en milieu basique

4 g du produit obtenu à l'exemple 3 sont dissous sous agitation dans 180 $cm^3$ de NaOH aqueux 0,7N. Le milieu réactionnel se colore en rose orangé au moment de l'addition. La coloration disparaît après 5 minutes de réaction. Le mélange est agité pendant 45 minutes. A la fin de la réaction, la solution basique est ajustée à 2 g pour 100 ml et dialysée contre de l'eau distillée dans un boyau (type WISKASE USA -seuil de coupure 6000 à 8000) jusqu'à pH neutre (2 jours environ). Le rétentat est ensuite concentré à 50 $cm^3$ par évaporation sous pression réduite, filtré sur membrane Millipore de 0,45µm puis lyophilisé. On récupère 3,1 g du sel de sodium du copolymère de formule V sous la forme d'une poudre brunâtre soluble dans l'eau, insoluble dans la plupart des solvants organiques. Sa masse moléculaire moyenne est 35000 (mesurée par chromatographie de perméation de gel, solvant : solution aqueuse de KBr 0,15M; étalons de polystyrène-sulfonate).

Le copolymère V sous forme acide est obtenu par percolation d'une solution aqueuse de son sel sur une résine Duolite C20MB sous forme $H^+$ et lyophilisation de l'éluat.

On obtient le copolymère sous forme d'une poudre blanche, soluble dans l'eau à tout pH, soluble dans le diméthylformamide, le diméthylsulfoxyde et le méthanol, mais insoluble dans les solvants chlorés, le tétrahydrofuranne, le dioxanne et l'acétone.

Le dosage des fonctions acides carboxyliques par NaOH 1M confirme la présence de deux fonctions acide par unité de répétition (masse d'un motif = 315).

Le spectre IR (pastille de KBr) présente trois bandes entre 1800 et 1500 $cm^{-1}$ caractéristiques d'un groupe acide carboxylique (1740 $cm^{-1}$) et d'un groupe amide (1650 et 1550 $cm^{-1}$).

Exemple 5 : Préparation des polymères à partir de la cystamine $H_2N-(CH_2)_2-S-S-(CH_2)_2-NH_2$ et du dichlorure de citrochloral.

2,14g de chlorhydrate de cystamine (9,5 × $10^{-3}$ mole), 5,44g de carbonate de sodium $10H_2O$ pour neutraliser et 0,4g de dodécylsulfate de sodium sont dissous dans 50 $cm^3$ d'un mélange benzène/eau (V/V; 60/40), sous forte agitation dans un mixeur de laboratoire; un introduit ensuite 3,74g de dichlorure de citrochloral (10,4 × $10^{-3}$ mole) dissous dans 30 $cm^3$ de benzène et 5,97g de carbonate de sodium (20,8 × $10^{-3}$ mole) dissous dans 20 $cm^3$ d'eau, de telle sorte que 90% des solutions soient ajoutées en 4 minutes, puis après une minute d'agitation, on ajoute les 10% restant en une minute; le polymère précipite dans le milieu réactionnel sous forme d'une pâte blanchâtre. En fin de réaction le milieu est acidifié jusqu'à pH2 par addition d'une solution aqueuse de HCl; le polycondensat brut est lavé à l'eau, puis séché sous vide à 50°C. On obtient 3g de polymère de qui comporte des groupes dioxolanne et imide. Ce produit est soluble dans les acides forts, dans la N-méthylpyrrolidone, le diméthylsulfoxyde et le diméthylformamide; il est insoluble dans l'eau, les alcools et les solvants chlorés.

Son spectre infrarouge est comparable à celui du composé de l'exemple 2-C, en ce qu'il présente les bandes caractéristiques du groupe imide cyclique à 1790 et 1710 $cm^{-1}$ et les bandes amides à 1650 et 1550 $cm^{-1}$.

Traité par une solution 0,7N aqueuse de NaOH, les cycles imides s'ouvrent et le polymère de formule V est soluble dans l'eau à pH7.

Exemple 6 : Préparation des polymères à partir de la L-cystine

L-cystine 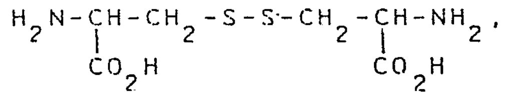

et du dichlorure de citrochloral.

a) Protection des 2 fonctions acides de la L-cystine : ditosylate de cystinate de dibenzyle.

On dissout 10g de L-cystine dans 43 ml d'alcool benzylique avec 17,4g d'acide para-toluène-sulfonique dans 100 ml de benzène. L'ensemble est amené à reflux du solvant dans un extracteur de type Dean-Stark jusqu'à fin d'élimination de l'eau.On introduit alors dans le milieu refroidi 50 ml de méthanol puis 500 ml d'éther éthylique. On isole ainsi 28,8 g de précipité, fondant à 169-171°C.

b) La polycondensation est effectuée comme dans l'exemple précédent avec 10,34g du ditosylate du diester benzylique de la L-cystine, 7,74g de $Na_2CO_3$, $10H_2O$ et 0,7g de dodécylsulfate de sodium dans 78 $cm^3$ d'un mélange $C_6H_6/H_2O$ (V/V-66/33), 5,57g de dichlorure de citrochloral dissout dans 52 $cm^3$ de benzène et 8,9g de $Na_2CO_3$, $10H_2O$ dans 26 $cm^3$ d'eau.

Après traitement du milieu réactionnel, on obtient 4,7g de polymère brut solide blanchâtre soluble dans les solvants chlorés ($CHCl_3$, $CH_2Cl_2$), le dioxanne, le tétrahydrofuranne et l'acétone et insoluble dans les alcools, l'eau et l'éther éthylique.

L'hydrolyse des fonctions esters benzyliques est effectuée par traitement du polymère par une solution à 33% de HBr dans l'acide acétique.

**Revendications**

1. Polyamides résultant de la condensation de l'acide citrique, par l'intermédiaire des groupes carboxyliques portés par des carbones en position 1 et 3, avec des diamines.

2. Polyamides selon la revendication 1 de formule

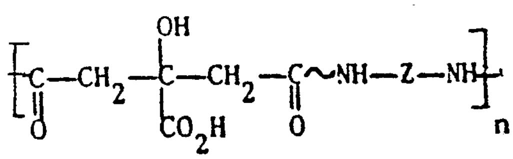

dans laquelle n représente un nombre entier inférieur à 1000, et Z représente une chaîne aliphatique linéaire ou ramifiée en $C_2$ à $C_{10}$, substituée ou non par les groupes hydrophiles -OH et -COOH, et dans laquelle peuvent être intercalés des atomes d'oxygène ou des atomes de soufre, ou Z représente une chaîne aryle ou alkylaryle.

et ∿ représente les deux types d'enchaînement lorsque Z est dissymétrique.

3. Polyamides selon la revendication 2 dans laquelle Z représente une chaîne aliphatique en $C_2$ à $C_8$.

4. Polyamides de formule

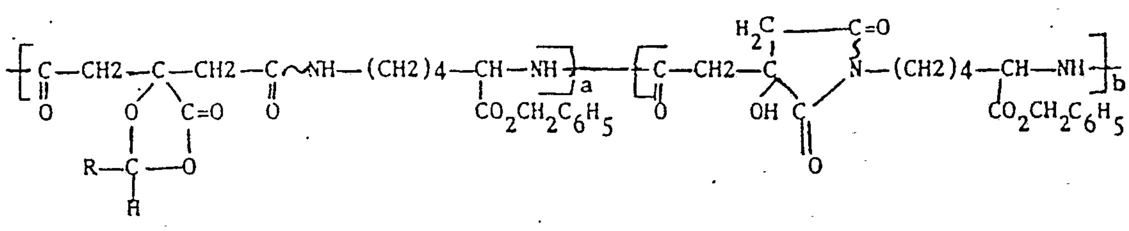

dans laquelle R représente $C_6H_5$, $CCl_3$, H ou un groupe alkyle en $C_1$ à $C_4$ et ∿ représente les deux types d'enchaînement dus à la diamine dissymétrique.

5. Polyamides de formule :

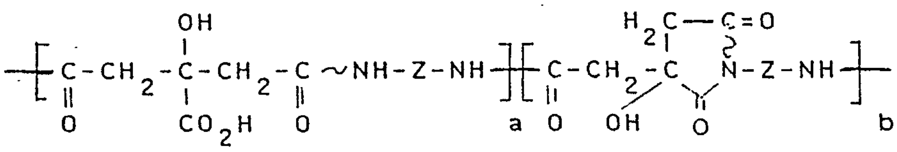

dans laquelle Z a la même signification que dans la revendication 2, a et b sont des nombres entiers, et ∿ représente les deux types d'enchaînement dus à la dissymétrie de la diamine.

6. Polyamides de formule $A_p$ - $B_q$ dans laquelle A représente :

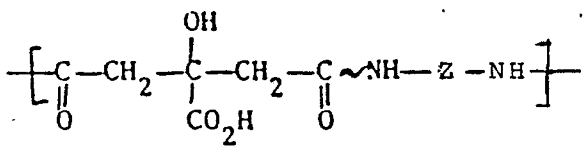

B représente :

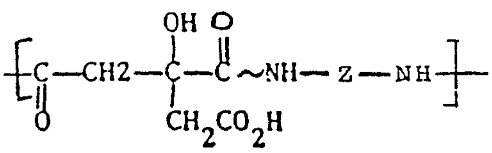

et p et q représentent des nombres entiers
et ∿ représente les deux types d'enchaînement dus à la dissymétrie de la diamine.

7. Procédé de préparation d'un polyamide selon la revendication 1, caractérisé en ce que l'on effectue une polycondensation de l'acide citrique, dont les groupes $CO_2H$ et OH portés par le carbone en position 2 ont été protégés, avec une diamine et en ce qu'on libère les groupes protégés.

8. Procédé selon la revendication 7 caractérisé en que l'acide citrique protégé mis en oeuvre répond à la formule

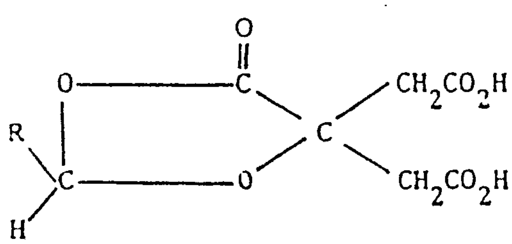

dans laquelle R représente H, un groupe alkyle en $C_1$ à $C_4$, $-CCl_3$ ou $-C_6H_5$.

9. Procédé selon l'une des revendications 7 et 8 caractérisé en ce que les groupes protégés sont libérés par hydrogénation catalytique.

10. Procédé selon l'une des revendications 7 à 9 caractérisé en ce que les groupes protégés sont libérés par l'action d'une base ou d'un acide en milieu aqueux.

11. Procédé de préparation selon l'une quelconque des revendications 7 à 10 d'un polyamide, dans lequel le groupe Z porte des groupes OH ou COOH, caractérisé en ce que ces groupes sont protégés avant la polycondensation.

12. Procédé selon la revendication 11 caractérisé en ce que le groupe COOH est protégé sous forme d'ester benzylique.

13. Application des polyamides selon les revendications 1 à 6 à la préparation de formes à libération contrôlée de médicaments.

14. Application des polyamides selon les revendications 1 à 6 à la préparation de prothèses chirurgicales ou de sutures et ligatures biodégradables.

15. Application des polyamides selon les revendications 1 à 6 à la préparation d'adhésifs biodégradables.

**REVENDICATIONS POUR L'ETAT CONTRACTANT SUIVANT: ES**

1. Procédé de préparation d'un polyamide résultant de la condensation de l'acide citrique, par l'intermédiaire des groupes carboxyliques portés par des carbones en position 1 et 3, avec des diamines, caractérisé en ce que l'on effectue une polycondensation de l'acide citrique, dont les groupes $CO_2H$ et OH portés par le carbone en position 2 ont été protégés, avec une diamine et en ce qu'on libère les groupes protégés.

2. Procédé selon la revendication 1, caractérisé en ce que l'on prépare un polyamide de formule

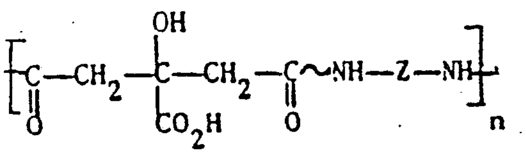

dans laquelle n représente un nombre entier inférieur à 1000, et Z représente une chaîne aliphatique linéaire ou ramifiée en $C_2$ à $C_{10}$, substituée ou non par les groupes hydrophiles -OH et -COOH, et dans laquelle peuvent être intercalés des atomes d'oxygène ou des atomes de soufre, ou Z représente une chaîne aryle ou alkylaryle
et ∿ représente les deux types d'enchaînement lorsque Z est dissymétrique.

3. Procédé selon la revendication 2, caractérisé en ce que l'on prépare un polyamide dans lequel Z représente une chaîne aliphatique en $C_2$ à $C_8$.

4. Procédé selon la revendication 3, caractérisé en ce que l'on prépare un polyamide de formule

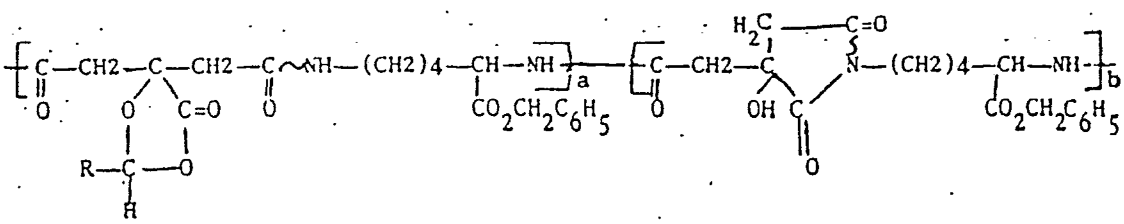

dans laquelle R représente $C_6H_5$, $CCl_3$, H ou un groupe alkyle en $C_1$ à $C_4$ et ∿ représente les deux types d'enchaînement dus à la diamine dissymétrique.

5. Procédé selon la revendication 4, caractérisé en ce que l'on prépare un polyamide de formule

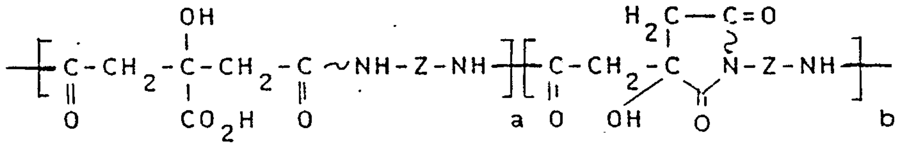

dans laquelle Z a la même signification que dans la revendication 2, a et b sont des nombres entiers, ∿ représente les deux types d'enchaînement dus à la dissymétrie de la diamine.

6. Procédé selon la revendication 5, caractérisé en ce que l'on prépare un polyamide de formule $A_p$ - $B_q$ dans laquelle A représente :

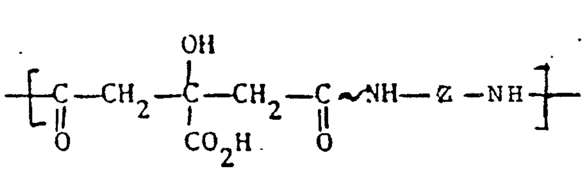

B représente :

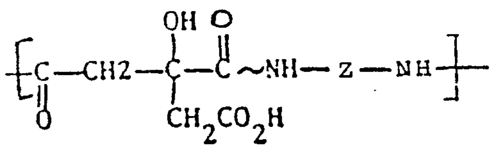

et p et q représentent des nombres entiers
et $\sim$ représente les deux types d'enchaînement dus à la dissymétrie de la diamine.

7. Procédé selon la revendication 1, caractérisé en ce que l'acide citrique protégé mis en oeuvre répond à la formule

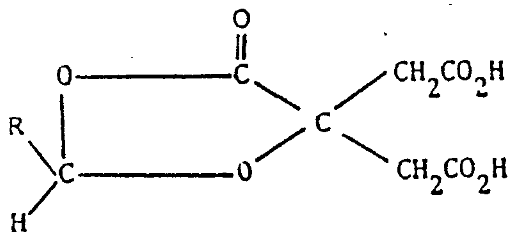

dans laquelle R représente H, un groupe alkyle en $C_1$ à $C_4$, $-CCl_3$ ou $-C_6H_5$.

8. Procédé selon la revendication 7, caractérisé en ce que les groupes protégés sont libérés par hydrogénation catalytique.

9. Procédé selon l'une des revendications 7 ou 8, caractérisé en ce que les groupes protégés sont libérés par l'action d'une base ou d'un acide en milieu aqueux.

10. Procédé de préparation selon l'une quelconque des revendications 1 à 9 d'un polyamide, dans lequel le groupe Z porte des groupes OH ou COOH, caractérisé en ce que ces groupes sont protégés avant la polycondensation.

11. Procédé selon la revendication 10, caractérisé en ce que le groupe COOH est protégé sous forme d'ester benzylique.

12. Application des polyamides obtenus par un procédé selon les revendications 1 à 11 à la préparation de formes à libération contrôlée de médicaments.

13. Application des polyamides obtenus par un procédé selon les revendications 1 à 11 à la préparation de prothèses chirurgicales ou de sutures et ligatures biodégradables.

14. Application des polyamides obtenus par un procédé selon les revendications 1 à 11 à la préparation d'adhesifs biodégradables.

Office européen des brevets

# RAPPORT DE RECHERCHE EUROPEENNE

Numero de la demande

EP 89 40 0635

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.4) |
| | Néant<br>----- | | C 08 G 69/26<br>A 61 K 9/22<br>A 61 L 17/00<br>A 61 L 25/00 |
| | | | DOMAINES TECHNIQUES RECHERCHES (Int. Cl.4) |
| | | | C 08 G<br>A 61 K<br>A 61 L |

Le présent rapport a été établi pour toutes les revendications

| Lieu de la recherche | Date d'achèvement de la recherche | Examinateur |
|---|---|---|
| LA HAYE | 06-06-1989 | LEROY ALAIN |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)